# EUROPÄISCHE PATENTANMELDUNG

(11) **EP 1 516 613 A1**
(43) Veröffentlichungstag der Anmeldung: **23.03.2005**
(21) Anmeldenummer: 04021734.1
(22) Anmeldetag: 13.09.2004
(51) Int. Cl.: A61K 7/075, A61K 7/02

(54) **Kosmetikum in einem Packmittel mit zwei Kammern.**

(30) Priorität: 12.09.2003 DE 10342211
(71) Anmelder: Beiersdorf AG, 20253 Hamburg (DE)
(72) Erfinder: Dörschner, Albrecht, 20146 Hamburg (DE); Argembeaux, Horst, 21465 Hamburg (DE); Detert, Marion, 22455 Hamburg (DE); Ruppert, Stephan, 20259 Hamburg (DE); Albrecht, Harald, 22083 Hamburg (DE); Küther, Jörg, 25469 Halstenbek (DE); Speiser, Fredy, 22301 Hamburg (DE)

(57) **Zusammenfassung**

Die vorliegende Erfindung betrifft ein Kosmetikum aus einem Packmittel mit mindestens zwei Kammern deren Inhalt mit Hilfe zweier gekoppelter Förderpumpen gleichzeitg über eine gemeinsame Austrittsöffnung entnommen werden kann und zwei kosmetischen Teilzubereitungen sowie die Verwendung des Packmittels.

## Beschreibung

Die vorliegende Erfindung betrifft ein Kosmetikum aus einem Packmittel mit zwei Vorratskammern und zwei kosmetischen Teilzubereitungen sowie die Verwendung des Packmittels.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde.

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV-Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsynthets) angeboten. Hautpflegeprodukte, in der Regel Cremes, Salben oder Lotionen, dienen meist der Befeuchtung und Rückfettung der Haut, denn die Aufgabe der Hautpflege ist es, den durch das tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die Art der Zusammensetzung kosmetischer Zubereitungen findet ihre natürlichen Grenzen, die durch die Verträglichkeit der Einzelkomponenten miteinander sowie der Stabilität von Einzelkomponenten im Trägermedium bestimmt werden. So sind beispielsweise Haut regenerierende Enzyme und Vitamine in kosmetischen Zubereitungen nicht unbegrenzt haltbar. Auch die Kombination unterschiedlich geladener Polymerer oder Tenside führt in kosmetischen Formulierungen zu Verklumpung und Ausfällungen.

Es hat nicht an Versuchen gefehlt, derartige Zubereitungen dem Verbraucher zugänglich zu machen. Meist werden dann Teilkomponenten der Formulierungen getrennt verpackt und aufbewahrt. In der Regel werden hierzu sogenannte Zwei-Kammer-Packmittel (engl. dual chamber) verwendet, bei denen die Teilzubereitungen in getrennten Vorratsgefäßen aufbewahrt und dem Packungsbehältnis gleichzeitig über eine gemeinsame Öffnung entnommen werden können. Derartige Zwei-Kammer-Packmittel werden beispielsweise in der WO 96/02230, der WO 96/37420, der WO 98/33477 und der EP 048703 beschrieben.

Herkömmliche Zwei-Kammer-Packmittel haben jedoch eine Reihe von Nachteilen, die ihre Einsatzmöglichkeiten im Kosmetikbereich bisher beschränkten.
■ Beide Teilzubereitungen müssen eine annähernd gleiche Viskosität aufweisen, um gleichmäßig aus dem Vorratsbehältnissen gefördert werden zu können. Sobald die Viskositätsunterschiede zu groß werden, wird kontinuierlich ein Überschussander niedrig-viskosen Zubereitung aus den Vorratsgefäßen gefördert. Am Ende bleiben große Mengen der höher viskosen Teilzubereitung unverbraucht im Packmittel zurück. Nach dem Stande der Technik lassen sich derartige Probleme allenfalls durch Zwei-Kammer-Packmittel mit zwei getrennten Entnahmeöffnungen lösen, wobei die Teilzubereitungen unvermischt bleiben.
■ Der Wirkstoffgehalt der Gesamtzubereitung lässt sich nicht individuell einstellen. Dies ist aber insbesondere dann erwünscht, wenn mehrere Anwender das gleiche Produkt benutzen (z.B. in Familien). Auch erweisen sich die unterschiedlichen Hautpartien als unterschiedlich empfindlich gegenüber Umwelteinflüssen (z.B. UV-Strahlung) oder unterschiedlich stark belastet (z.B. starke Verschmutzung der Hände).

Es war daher die Aufgabe der vorliegenden Erfindung, die Mängel des Standes der Technik zu beseitigen. Ferner war es die Aufgabe der vorliegenden Erfindung, ein Kosmetikum zu entwickeln, welches die simultane Anwendung an sich unverträglicher Inhaltsstoffe von Kosmetika und Dermatika ermöglicht. Ferner sollten kosmetische bzw. dermatologische Teilzubereitungen mit stark unterschiedlicher Viskosität dem System in gleichmäßiger Zusammensetzung nahezu vollständig entnehmbar sein. Nicht zuletzt war es die Aufgabe der vorliegenden Erfindung, ein von dem Verbraucher einfach zu handhabendes Kosmetikum zur Verfügung zu stellen, mit welchem er problemlos auf seine (wechselnden) individuellen Bedürfnisse zugeschnittene kosmetische oder dermatologische Wirkstoffkonzentrationen entnehmen kann.

Die Aufgaben werden überraschend gelöst durch ein Kosmetikum, welches gebildet wird aus
a) einem Packmittel mit zwei von einander getrennten Vorratskammern, deren Inhalt mit Hilfe zweier gekoppelter Förderpumpen gleichzeitig über eine gemeinsame Austrittsöffnung entnommen werden kann und dadurch gekennzeichnet ist, dass sich das Mengenverhältnis der durch die beiden Pumpen geförderten kosmetischen Zubereitungen zueinander mit Hilfe eines Regelmechanismus frei einstellen lässt, sowie
b) zwei kosmetischen Zubereitungen 1 und 2, die sich jeweils in einer der beiden Vorratskammern des Packmittels befinden und dadurch gekennzeichnet sind, dass sie sich in ihrer Viskosität und/oder Konzentration an kosmetischen und/oder dermatologischen Wirkstoffen von einander unterscheiden.

Ferner werden die Aufgaben für den Fachmann unerwartet gelöst durch die Verwendung eines Packmittel mit zwei von einander getrennten Vorratskammern, deren Inhalt mit Hilfe zweier gekoppelter Förderpumpen gleichzeitig über eine gemeinsame Austrittsöffnung entnommen werden kann und dadurch gekennzeichnet ist, dass sich das Mengenverhältnis der durch die beiden Pumpen geförderten kosmetischen Zubereitungen zueinander mit Hilfe eines Regelmechanismus frei einstellen lässt, zur variablen Einstellung des Mischungsverhältnisses zweier kosmetischer Zubereitungen unterschiedlicher Viskosität.

Die Aufgaben werden überraschend ferner gelöst durch die Verwendung eines Packmittels mit zwei von einander getrennten Vorratskammern, deren Inhalt mit Hilfe zweier gekoppelter Förderpumpen gleichzeitig über eine gemeinsame Austrittsöffnung entnommen werden kann und dadurch gekennzeichnet ist, dass sich das Mengenverhältnis der durch die beiden Pumpen geförderten kosmetischen Zubereitungen zueinander mit Hilfe eines Regelmechanismus frei einstellen lässt, zur Steigerung der Wirksamkeit kosmetischer Zubereitungen und der in ihnen enthaltenen Wirkstoffe, welche durch die getrennte Aufbewahrung einzelner Teilzubereitungen und deren gleichzeitiger Applizierung in variabler Dosierung bewirkt wird.

Auch werden die der Erfindung zugrunde liegenden Aufgaben überraschend gelöst durch die Verwendung eines Packmittels mit zwei von einander getrennten Vorratskammern, deren Inhalt mit Hilfe zweier gekoppelter Förderpumpen gleichzeitig über eine gemeinsame Austrittsöffnung entnommen werden kann und dadurch gekennzeichnet ist, dass sich das Mengenverhältnis der durch die beiden Pumpen geförderten kosmetischen Zubereitungen zueinander mit Hilfe eines Regelmechanismus frei einstellen lässt, zur individuellen Wirkstoffdosierung wirkstoffhaltiger kosmetischer oder dermatologischer Zubereitungen.

Bei den Zubereitungen 1 und 2 handelt es sich dabei erfindungsgemäß um Teilzubereitungen (Teilkomponenten) einer kosmetischen Gesamtzubereitung, welche die vom Verbraucher angewendete Zubereitung darstellt.

Erfindungsgemäß vorteilhaft kann das erfindungsgemäße bzw. erfindungsgemäß verwendete Packmittel auch mehr als zwei Kammern aufweisen und in einem solchen Fall mehr als zwei unterschiedliche kosmetische (bzw. dermatologische) Zubereitungen enthalten.

Vorteilhafte Packmittel im Sinne der vorliegenden Erfindung sind dadurch gekennzeichnet, dass die variable Einstellung des Mengenverhältnisses dadurch erfolgt, däss der mit Hilfe eines gemeinsamen Pumpauslösers erzeugte Druck a uf die beiden Förderpumpen mit Hilfe einer drehbaren Scheibe unterschiedlich stark auf die beiden Pumpenköpfe verteilt wird. Die beiden Vorratsgefäße können einzeln von einander getrennt oder fest miteinander verbunden sein. Ferner kann es vorteilhaft im Sinne der vorliegenden Erfindung sein, dass sich die einzelnen oder verbundenen Vorratsbehälter auswechseln oder nachfüllen lassen.

Bei schäumbaren Zubereitungen kann zum Aufschäumen jede einzelne Fördereinheit mit einem eigenen Aufschäumsieb oder beide Fördereinrichtungen mit einem gemeinsamen Aufschäumsieb ausgestattet sein. Entsprechend befindet sich die Mischungskammer für die beiden Zubereitungen 1 und 2 vor oder hinter den Sieben/dem Sieb angeordnet.

Das Packmittel kann ganz oder teilweise transparent oder transluzent gestaltet sein, wodurch die die Teilzubereitungen (Zubereitung 1 und 2) ganz oder teilweise sichtbar werden und sich vorteilhaft mit Schweb-und/oder Effektstoffen optisch attraktiv gestalten lassen.

Bei vorteilhaften Packmitteln im Sinne der vorliegenden Erfindung sind die beiden Kammern des Packmittels über ein Verbindungssystem so aneinander montierbar, dass sie bei der Anwendung zusammen mit dem Dosierkopf eine Einheit bilden, sich aber durch einfaches Zerlegen auch einzeln ersetzen bzw. nachfüllen lassen. Vorzugsweise sind die Kammern sowohl aneinander befestigt als auch durch Aufsetzen des Dosierkopfes fixiert. Vorzugsweise werden beide Kammern identisch geformt sein, um eine variable Kombination zu ermöglichen.

Besonders vorteilhafte Packmittel im Sinne der vorliegenden Erfindung stellen die Zwei-Kammer-Packmittel der Firma Versadial™ (Versadial dual dispenser bottle) dar.

Vorteilhaft im Sinne der vorliegenden Erfindung ist es, wenn der Viskositäts-Unterschied zwischen den beiden kosmetischen Zubereitungen 1 und 2 größer als 50 mPa s und kleiner als 3000 mPa s und bevorzugt, wenn der der Viskositäts-Unterschied zwischen den beiden kosmetischen Zubereitungen 1 und 2 größer als 100 mPa s und kleiner als 2000 mPa s beträgt.

Vorteilhaft im Sinne der vorliegenden Erfindung beträgt die Viskosität der höher-viskosen Teilzubereitung maximal 10000 mPa s mPas und bevorzugt maximal 7000 mPas.

Vorteilhaft im Sinne der vorliegenden Erfindung beträgt die Viskosität der Teilzubereitung mit der niedrigeren Viskosität mindestens 300 mPas und bevorzugt mindestens 1000 mPas.

Die Viskosität der beiden Zubereitungen 1 und 2 lässt sich dabei vorteilhaft durch den Gehalt an einem oder mehreren Verdickern (auch Gelbildner oder Hydrokolloide genannt) regulieren. Als Verdickungsmittel im Sinne der vorliegenden Erfindung können vorteilhaft eingesetzt werden:
■ organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
■ organische, abgewandelte Naturstoffe, wie z. B. Carboxymethylcellulose und andere Celluloseether, Hydroxyethyl- und -propylcellulose und dergleichen,
■ organische, vollsynthetische Verbindungen, wie z. B. Polyacryl- und Polymethacryl-Verbindungen, Vinylpolymere, Polycarbonsäuren, Polyether, Polyimine, Polyamide,
■ anorganische Verbindungen, wie z. B. Polykieselsäuren, Tonmineralien wie Montmorillonite, Zeolithe, Kieselsäuren.

Erfindungsgemäß bevorzugte Verdicker sind beispielsweise Methylcellulosen, als welche die Methylether der Cellulose bezeichnet werden. Sie zeichnen sich durch die folgende Strukturformel aus 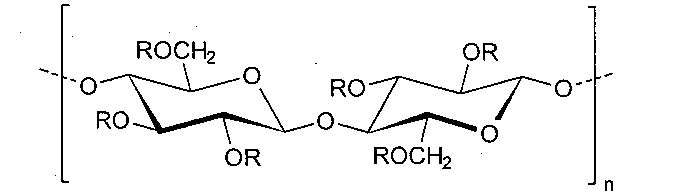 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders b evorzugt s ind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird.

Es wird von Xanthomonas campestris und einigen anderen Species unter aeroben Bedingungen mit einem Molekulargewicht von 2×106 bis 24×106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure; Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-Lgalactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute 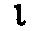 -Carrageenan ist sowohl wasserlöslich als auch gelbildend. Auch die Art vorhandener Kationen (K+, NH4+, Na+, Mg2+, Ca2+) beeinflußt die Löslichkeit der Carrageene.

Polyacrylate sind ebenfalls vorteilhaft im Sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

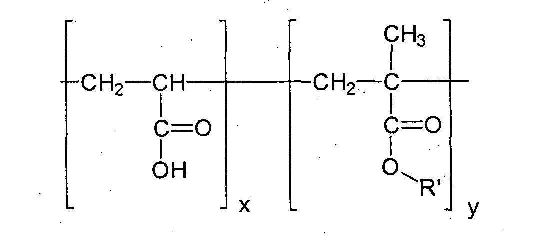

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der N ational Starch erhältlich) und ähnliche Polymere.

Die Gesamtmenge an einem oder mehreren Hydrokolloiden wird in den fertigen kosmetischen oder dermatologischen Zubereitungen vorteilhaft kleiner als 1,5 Gew.-%, bevorzugt zwischen 0,1 und 1,0 Gew.-%, bezogen auf das Gesamtgewicht der Zubereitungen, gewählt.

Es ist beispielsweise vorteilhaft im Sinn der vorliegenden Erfindung, wenn der Gehalt an einem oder mehreren Polyacrylaten in der kosmetischen oder dermatologischen Teilzubereitungen aus dem Bereich von 0,5 bis 4 Gew.-%, ganz besonders vorteilhaft von 0,7 bis 2 Gew.-% gewählt wird, jeweils bezogen auf das Gesamtgewicht der Teilzubereitung.

Es ist vorteilhaft im Sinne der vorliegenden Erfindung, wenn der Konzentrationsunterschied an einem oder mehreren bestimmten (jeweils gleichen) kosmetischen und/oder dermatologischen Wirkstoffen in den kosmetischen Zubereitungen 1 und 2 mindestens 0,01 % beträgt.

Es ist vorteilhaft im Sinne der vorliegenden Erfindung, wenn die kosmetische Zubereitung 1 einen oder mehrere Inhaltsstoffe enthält, die aus der Gruppe der anionischen Tenside, nichtionischen Tenside, amphoteren Tenside, Betaine, ethoxylierten Mono-, di- und Triglyceriden, anionischen, nichtionischen und amphoteren Polymeren, Parfümölen, Enzymsubstraten, Konditionierer, Emulgatoren Lipiden und Silikonölen gewählt werden können.

Ferner ist es vorteilhaft im Sinne der vorliegenden Erfindung, wenn die kosmetische Zubereitung 2 einen oder mehrere Inhaltsstoffe enthält, die aus der Gruppe der wasserlöslichen Wirkstoffe, kationischen Tenside, kationischen Poymeren, Emulgatoren und Farbstoffen gewählt werden können.

Vorteilhaft im Sinne der vorliegenden Erfindung sind beispielsweise die folgenden Kombinationen aus Teilzubereitungen:

| **Kammer 1** | **Kammer 2** |
|---|---|
| Shampoo | Haarkur |
| Duschzubereitung | Shampoo |
| Wässrige, tensidhaltige Zubereitung | Zubereitung die sich bei Kontakt mit Wasser erwärmt |
| Tensidhaltige Zubereitung | Emulsion |
| Tensidhaltige Zubereitung | Zubereitung mit Effektstoffen |
| Gelförmiges Haarfrisiermittel ("Styling Gel") | Zubereitung mit Effektstoffen |
| Tensidhaltige Zubereitung | Carbonat-haltige Phase |

Die Teilzubereitungen in den Kammern 1 und 2 des Schaumdispensers enthalten vorteilhaft im Sinne der vorliegenden Erfindung kosmetische Wirk-, Hilfs- und Zusatzstoffe. Diese können beispielsweise aus den folgenden kosmetischen Oder dermatologischen Inhaltsstoffen gewählt werden:

### Tenside

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

### Filmbildner

Erfindungsgemäß vorteilhafte Filmbildner können dabei aus den in der Tabelle aufgelisteten Verbindungen gewählt werden.

| **Bezeichnung nach INCI** | **CAS-Nummer** | **Polymertyp** | **Beispiel (Handelsname)** |
|---|---|---|---|
| Polyquaternium-2 | CAS 63451-27-4 | Urea, N, N'- bis[3-(dimethylamino)propyl]- , polymer mit 1, 1'-oxybis(2- chloroethan) | Mirapol® A-15 |
| Polyquaternium-5 | CAS 26006-22-4 | Acrylamid, β-Methacryloxyethyltriethyl ammoniummethosulfat | |
| Polyquaternium-6 | CAS 26062-79-3 | N,N-Dimethyl-N-2-propenyl-2-propen-aminiumchlorid | Merquat® 100 |
| Polyquaternium-7 | CAS 26590-05-6 | N,N-Dimethyl-N-2-propenyl-2-propen-aminiumchlorid, 2-Propenamid | Merquat@ S |
| Polyquaternium-10 | CAS 53568-66-4, 55353-19-0, 54351-50-7, 68610-92-4, 81859-24-7 | Quaternäres Ammoniumsalz der Hydroxyethylcellulose | Celquat® SC-230M |
| Polyquaternium-11 | CAS 53633-54-8 | Vinylpyrrolidon/dimethyla minoethyl-Methacrylat-Copolymer/Diethylsulfat-Reaktionsprodukt | Gafquat®755N |
| Polyquaternium-16 | CAS 29297-55-0 | Vinylpyrrolidon/vinylimid azolinummethochlorid-Copolymer | Luviquat® HM552 |
| Polyquaternium-17 | CAS 90624-75-2 | | Mirapol®AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinyloctadecylether | |
| Polyquaternium-21 | | Polysiloxanpolydimethyl-dimethylammoniumacetat-Copolymer | Abil® B 9905 |
| Polyquaternium-22 | CAS 53694-17-0 | Dimethyldiallylammoniumchlorid/Acrylsäure-Copolymer | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacryl amidopropyltrimethylammoniumchlorid-Copolymer | Gafquat®HS-100 |
| Polyquaternium-29 | CAS 92091-36-6, 148880-30-2 | Chitosan, das mit Propylenoxid umgesetzt u. mit Epichlorhydrin quaternisiert wurde | Lexquat® CH |
| Polyquaternium-31 | CAS 136505-02-7, 139767-67-7 | Polymeres, quaternäres Ammoniumsalz, das durch die Umsetzung von DMAPA-Acrylate/Acrylsäure/Acryl onitrogens-Copolymeren u. Diethylsulfat hergestellt wird | Hypan® QT 100 |
| Polyquaternium-32 | CAS 35429-19-7 | N,N,N-Trimethyl-2-{[82-methyl-1-oxo-2-propenyl)oxy]ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |
| Erfindungsgemäß vorteilhafte Filmbildner | | | |

Weitere erfindungsgemäß vorteilhafte Filmbildner stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Auch n ichtionische P oly-N-vinylpyrrolidon/Polyvinylacetat-Copolymere (z.B. L uviskol V A 64W®, BASF), anionische Acrylat-Copolymere (z.B. Luviflex soft®, BASF), und/oder amphotere Amid/Acrylat/Methacrylat Copolymere (z.B. Amphomer®, National Starch) können erfindungsgemäß vorteilhaft als Filmbildner eingesetzt werden.

### Komplexbildner

Es ist auch von Vorteil, den erfindungsgemäßen Zubereitungen Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexanteträessigsäure (CDTA) und deren Anionen, Tetrasodium Iminodisuccinate, Trisodium Etylenediamine Disuccinate.

### Ölkomponenten/Lipide

Eine gegebenenfalls gewünschte Ölkomponente der kosmetischen oder dermatologischen Teilzubereitungen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der Vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 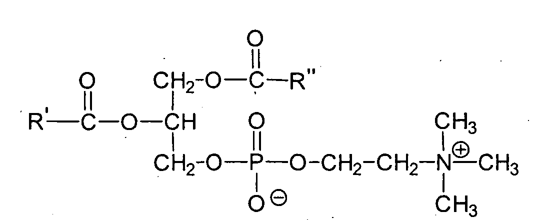 auszeichnen, wobei R' und R" typischerweise unverzweigte aliphatische Reste mit 15 oder 17 Kohlenstoffatomen und bis zu 4 cis-Doppelbindungen darstellen.

### Wässrige Phase

Neben Wasser kann/können der/die erfindungsgemäßen Phasen auch wasserlösliche Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Erfindungsgemäß vorteilhaft kann mindestens eine der erfindungsgemäßen Zubereitungen 1 und 2 neben einer oder mehreren Wasserphasen eine oder mehrere Ölphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine P IT-Emulsion), eine F eststoff-Emulsionen (d. h. eine E mulsion, welche durch Feststoffe stabilisiert ist, z. B. eine Pickering-Emulsion) sein, wobei transparente oder transluzente Mikroemulsionen erfindungsgemäß besonders bevorzugt sind.

### Wirkstoffe/Aktivstoffe

Als Wirk- und/oder Aktivstoffe im Sinne der vorliegenden Erfindung können beispielsweise eingesetzt werden: kosmetische UV-Lichtschutzfilter, Enzyme (oder deren Derivate), Vitamine oder deren Derivate, Antioxidantien, Pflanzenextrakte und dergleichen mehr.

### Antioxidaniten

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. a-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin A, D, F und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20Gew.-%, insbesondere 0,1 bis 10 Gew.-%, bezogen auf das Gesamtgewicht der Teilzubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Teilzubereitung, zu wählen.

### UV-Lichtschutzfilter

Erfindungsgemäß vorteilhafte UV-Lichtschutzfilter sind sowohl anorganische Pigmente als auch organische Filtersubstanzen.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Die erfindungsgemäßen Titandioxide zeichnen sich durch eine Primärpartikelgröße zwischen 10 nm bis 150 nm aus.

Zinkoxide können im Sinne der vorliegenden Erfindung auch in Form kommerziell erhältlicher öliger oder wässriger Vordispersionen zur Anwendung kommen. Erfindungsgemäß g eeignete Zinkoxidpartikel und Vordispersionen von Zinkoxidpartikeln zeichnen sich durch eine Primärpartikelgröße von < 300 nm aus.

Die Gesamtmenge an einem oder mehreren anorganischen Pigmenten i n der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Weitere vorteilhafte UV-A-Filtersubstanzen sind Hydroxybenzophenone, die sich durch die folgende Strukturformel auszeichnen: 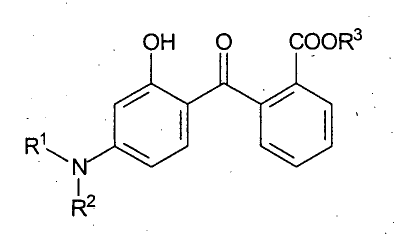 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welches unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 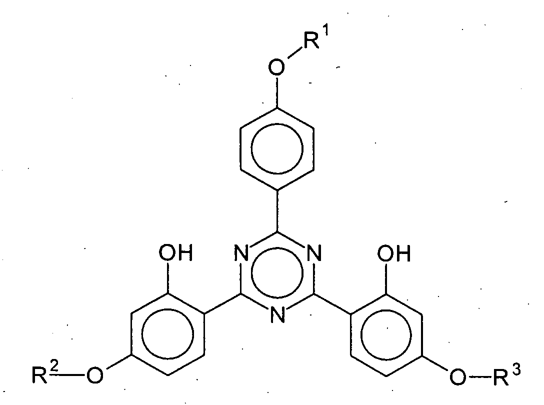 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 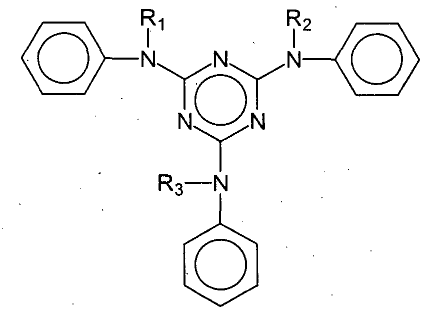 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 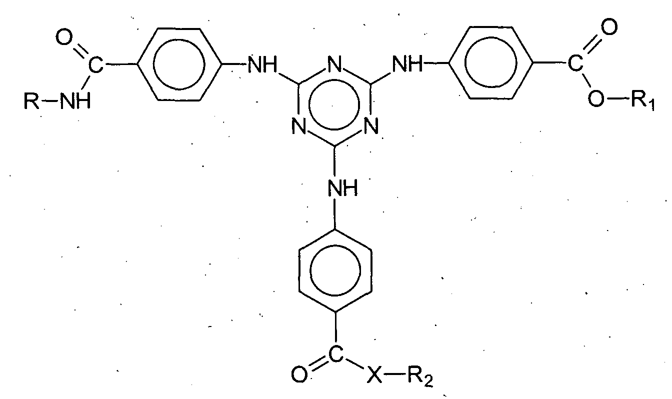 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 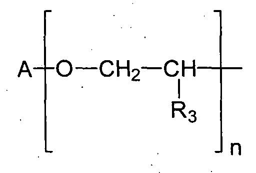 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 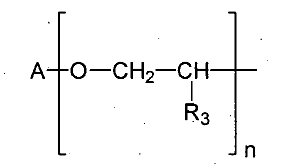 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt, wenn X ein Sauerstoffatom darstellt.

Besonders bevorzugte UV-Filtersubstanz im Sinne der vorliegenden Erfindung ist ferner ein unsymmetrisch substituiertes s-Triazin, welches auch als Dioctylbutylamidotriazon (INCI: Dioctylbutamidotriazone) bezeichnet wird und unter der Handelsbezeichnung UVA-SORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 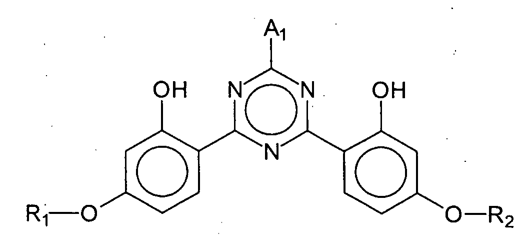 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxyl-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.
Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCl: Homosalate), 2-Ethylhexyl-2-hydroxybenzoat (2-Ethylhexylsalicylat, Octylsalicylat, INCl: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCl: Octyl Methoxycinnamate) und 4-Methoxyzimtsäureisopentylester (Isopentyl-4-methoxycinnamat, INCl: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: D imethicodiethylbenzalmalonat) welches beispielsweise unter der Handelsbezeichnung Parsol® SLX bei Hoffmann La Roche erhältlich ist.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

### Vitamine und Enzyme

Es ist erfindungsgemäß vorteilhaft, wenn in der Zubereitung 1 als Wirkstoffe, die in derivatisierter Form vorliegen, Vitamine eingesetzt werden. Erfindungsgemäß bevorzugt werden dabei Retinylpalmitat, Ascorbylglucosid, Tocopherylacetat, Tocopherylpalmitat eingesetzt. Erfindungsgemäß besonders bevorzugt ist der Einsatz Retinylpalmitat.

Erfindungsgemäß vorteilhaft werden ein oder mehrere kosmetische und/oder dermatologische Wirkstoffe, die in Form von stabilen Derivaten vorliegen, in einer Gesamtkonzentration von 0,01 bis 10 Gewichts- %, bevorzugt in einer Konzentration 0,01 von bis 5 Gewichts- % und ganz besonders bevorzugt in einer Konzentration von 0,05 bis 1 Gewichts- %, jeweils bezogen auf das Gesamtgewicht der Gesamtzubereitung, einer der Teilzubereitungen zugefügt.

Erfindungsgemäß vorteilhaft ist der Einsatz von Retinylpalmitat in einer Konzentration von 0,05 bis 1 Gewichts- %, bevorzugt in einer Konzentration von 0,01 bis 0,5 Gewichts-% und ganz besonders bevorzugt in einer Konzentration von 0,05 bis 0,2 Gewichts-%, jeweils bezogen auf das Gesamtgewicht der Teilzubereitung.

Das erfindungsgemäße Kosmetikum und/oder Dermatikum enthält vorteilhaft in der zweiten Teilzubereitung als Enzyme Lipasen, Esterasen, Proteasen und andere Hydrolasen. Dabei sind Lipasen erfindungsgemäß bevorzugt.

Als erfindungsgemäße Enzyme können vorteilhaft Enzymextrakte aus *Alcaligenes sp.* (z.B. von der Firma Amano Enzyme Europe Ltd.), *Aspergillus niger* (z.B. von den Firmen Amano Enzyme Europe Ltd. oder Fluka), *Candida cylindracea* (z.B. von den Firmen Sigma, Aldrich, Fluka, Amano Enzyme Europe Ltd., Boeringer-Mannheim), *Candida* lipolytica ( z.B. von d en F irmen A mano E nzyme E urope L td., F luka), *Chromobacterium viscosium* (z.B. von den Firmen Sigma), *Humicola laguninosa* (z.B. von der Firma Amano Enzyme Europe Ltd.), *Mucor miehei* (z.B. von den Firmen Amano Enzyme Europe Ltd., Novo), *Penecillium* roqueforti (z.B. von der Firma Fluka), *Porcine pancreas* (z.B. von den Firmen Sigma, Aldrich, Fluka, Amano Enzyme Europe Ltd., Boeringer-Mannheim), *Pseudomonas aegruginosa* (z.B. von der Firma Amano Enzyme Europe Ltd.), *Pseudomonas* fluorescens (z.B. von den Firmen Amano Enzyme Europe Ltd., Fluka), *Pseudomonas sp*.(z.B. von den Firmen Sigma, Boeringer-Mannheim), *Rhizopus delemar* (z.B. von der Firma Sigma, Fluka, Boeringer-Mannheim), *Rhizopus japonicus* (z.B. von den Firmen Amano Enzyme Europe Ltd.), *Rhizopus oryzae* (z.B. von der Firma Amano Enzyme Europe Ltd.), *Rhizopus sp.* (z.B. von den Firmen Amano Enzyme Europe Ltd., Serva) und/oder Weizenkeim (z.B.von der Firma Sigma) eingesetzt werden.

Dabei sind als Enzym-Extrakte Lipasen aus Schweinepankreas (*Porcine pancreas*), *Aspergillus niger, Chromobacterium viscosium, Geotrichum candidum, Penecillium camberti, Penecillium roqueforti* erfindungsgemäß bevorzugt.

Erfindungsgemäß ganz besonders bevorzugt sind Lipasen der Art *Mucor miehei* (z.B. Lipozyme® von der Firma Novo Nordisk ), *Humicola laguninosa, Pseudomonas aegruginosa, Pseudomonas fluorescens, Pseudomonas sp., Candida cylindracea* und/oder *Candida lipolytica.*

Erfindungsgemäß vorteilhaft liegt die zweite Teilzubereitung in Form einer wässrigen Lösung vor. Deren Gehalt an einem oder mehreren Enzymen beträgt erfindungsgemäß vorteilhaft von 0,1 bis 5 Gewichts- %, bevorzugt von 1 bis 5 Gewichts- % und ganz besonders bevorzugt von 1 bis 2 Gewichts-%, jeweils bezogen auf das Gesamtgewicht der Teilzubereitung.

Erfindungsgemäß vorteilhaft beträgt die Konzentration an Lipasen aus *Humicola laguninosa* von 0,1 bis 5 Gewichts- %, bevorzugt von 1 bis 5 Gewichts- % und ganz besonders bevorzugt von 1 bis 2 Gewichts- %, jeweils bezogen auf das Gesamtgewicht der Teilzubereitung.

Erfindungsgemäß vorteilhaft beträgt die Konzentration an Lipasen aus *Mucor miehei* von bis Gewichts-%, bevorzugt von 0,1 bis 5 Gewichts- %, bevorzugt von 1 bis 5 Gewichts- % und ganz besonders bevorzugt von 1 bis 2 Gewichts- %, jeweils bezogen auf das Gesamtgewicht der Teilzubereitung.

Erfindungsgemäß vorteilhaft beträgt die Konzentration an Lipasen aus *Pseudomonas aegruginosa* von 0,1 bis 5 Gewichts- %, bevorzugt von 1 bis 5 Gewichts- % und ganz besonders bevorzugt von 1 bis 2 Gewichts- %, jeweils bezogen auf das Gesamtgewicht der Teilzubereitung.

Die Wirkstoffe und Enzyme können ganz oder teilweise verkapselt sein. Dabei können erfindungsgemäß vorteilhaft sowohl Lipidpartikel als auch Cyclodextrine und/oder deren Derivate zur Verkapselung verwendet werden.

Erfindungsgemäß vorteilhaft kann mindestens eine der beiden Teilzubereitungen 1 und 2 in Form einer Emulsion oder Dispersion vorliegen, wobei sowohl Makro- als auch Mikroemulsioonen, O/W-Emulsionen, W/O-Emulsionen, S/W-Emulsionen, W/S-Emulsionen als auch multiple Emulsionen vorteilhaft im sinne der vorliegenden Erfindung eingesetzt werden können.

Die Zusammensetzungen enthalten gemäß der Erfindung außer den vorgenannten Substanzen gegebenenfalls weitere in der Kosmetik üblichen. Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Selbstbräuner (z.B. DHA), Depigmentiermittel, Antischuppenwirkstoffe, Vitamine, weitere Wirkstoffe, Komplexe aus gamma-Oryzanol und Calciumsalzen, Niacinamid und dessen Derivate, Panthenol und dessen Derivate, Subtilisin, Mineralien, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Es ist erfindungsgemäß vorteilhaft, wenn die beiden Teilzubereitungen 1 und 2 unterschiedlich gefärbt und/oder mit unterschiedlichen Effektstoffen beladen sind.

Erfindungsgemäß ist die Verwendung des erfindungsgemäßen Kosmetikums und/oder Dermatikums zur Reinigung und Pflege der Haut und/oder Hautanhangsgebilde.

Insbesondere ist die Verwendung des erfindungsgemäßen Kosmetikums und/oder Dermatikums als Duschgel, Wannenbad oder Haarwaschmittel (Haarshampoo) erfindungsgemäß.

Doch ist auch die Verwendung des erfindungsgemäßen Kosmetikums und/oder Dermatika als Hautcreme, Lotion oder Gel zur Pflege der Haut erfindungsgemäß vorteilhaft.

Ferner eignen sich die erfindungsgemäßen Produkte hervorragend zur Reinigung und Pflege von Gegenständen des täglichen Lebens (z.B. Geschirr, Tisch- und Schrankflächen, Autos, Kleidungsstücke).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1.

### Kammer 1: Shampoo ; Kammer 2: Haarkur:

### Kammer 1 (Shampoo): Beispiele 1-5

| | **1** | **2** | **3** | **4** | **5** |
|---|---|---|---|---|---|
| Natrium Laurylethersulfat | 9,0% | 9,0%. | 10,0% | 8,0% | 7,5% |
| Cocamidopropyl Betain | 4,0% | 3,5% | 3,0% | 4,5% | 4,0% |
| Dinatrium PEG-5 Laurylcitrat Sulfosuccinat | 3,5% | 3,0% | 2,5% | 4,5% | 3,0% |
| PEG-200 hydriertes Glycefyl Palmat | 0.1 % | 0.1 % | 0.1 % | 0.2% | 0,3% |
| Polyquaternium-10 | 0.3% | 0.1% | 0.1% | 0.3% | - |
| Guar Hydroxypropyl-Trimonium Chlorid | - | - | 0.1 % | 0.2% | 0.2% |
| Perlglanz | 1.5% | 3% | 4% | 2% | 2,5% |
| Trübungsmittel | - | - | - | - | 0.5% |
| Iminodibersteinsäure | 0.1% | 0.2% | 0.1% | 0.5% | 0.5% |
| PEG-40 hydriertes Rizinusöl | 0.2% | 0.4% | 0.2% | 0.2% | 0.2% |
| Konservierungsmittel | 0.8% | 0.8% | 0.8% | 0.8% | 0.8% |
| Natriumchlorid | 0,9% | 1,0% | 1,2% | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |
| Der pH-Wert wird auf 3,5 eingestellt. | | | | | |

### Kammer 2 (Haarkur): Beispiele 1-5

| | **1** | **2** | **3** | **4** | **5** |
|---|---|---|---|---|---|
| Hydroxypropylmethylcellulose | 0,5% | 0,5% | 0,75% | 1,0% | 0,5 |
| Glycerin | 3,0% | 3,0% | 3,5% | 2,5% | 2,0% |
| Cetearylalkohol | 2,5% | 1,5% | 2,75% | 3,5% | 2,5% |
| Glycerylstearat | 2,0% | 2,0% | 2,0% | 1,5% | 2,5% |
| Calciumlactat | 0,50% | 0,1% | 0,02% | 0,8% | 0,9% |
| Konservierungsmittel | 0,65% | 0,65% | 0,65% | 0,65% | 0,65% |
| Verdicker | q.s. | q.s. | q.s. | q.s. | q.s. |
| Parfüm, pH-Einstellung | q.s. | q.s. | q.s. | q.s. | q.s. |
| Parfum | q.s. | q.s. | q.s. | q.s. | q.s. |
| Wasser | ad 100 | ad 100 | ad 100 | ad 100 | ad 100 |

### Kammer 2 (Haarkur):Beispiele 6 - 10

| | **6** | **7** | **8** | **9** | **10** |
|---|---|---|---|---|---|
| Hydroxypropylmethylcellulose | 0,2% | 0,2% | 0,2% | 0,1% | 0,3 |
| Glycerin | 3,0%, | 3,0% | 3,5% | 1,5% | 2,0% |
| Cetearylalkohol | 2,5% | 2,5% | 2,5% | 1,5% | 3,5% |
| Glycerylstearat | 2,0% | 2,0% | 2,0% | 1,5% | 2,5% |
| Calciumlactat | 0,50% | 0,1% | 0,02% | 0,7% | 0,4% |
| Konservierungsmittel | 0,65% | 0,65% | 0,65% | 0,65% | 0,65% |
| Verdicker | q.s. | q.s. | q.s. | q.s. | q.s. |
| Parfüm, pH-Einstellung | q.s. | q.s. | q.s. | q.s. | q.s. |
| Parfum | q.s. | q.s. | q.s. | q.s. | q.s. |
| Wasser | ad 100 | ad 100 | ad 100 | ad 100 | ad 100 |
| Der pH-Wert wird auf 3,0 eingestellt. | | | | | |

### Beispiel 2:

### Kammer 1: Duschformulierung; Kammer 2: Shampoo

### Kammer 1 (Duschformulierung) Beispiele 1-5:

| | **1** | **2** | **3** | **4** | **5** |
|---|---|---|---|---|---|
| Natrium Laurethsulfat | 13,2% | 11 % | 9,5% | 12,0% | 8,7% |
| Cocoamidopropylbetain | 1,65% | 3,3% | 3,8% | 4,5% | 2,5% |
| Natriumcocoylglutamat | 1,25% | 0,75% | 2,5% | 1,5% | 1,75% |
| PEG-40 hydriertes Rizinusöl | 0,50% | 0,50% | 0,50% | 0,50% | 0,50% |
| PEG-100 hydriertes Glycerylpalmitat | 0,50% | 0,50% | 0,50% | 0,65% | 0,50% |
| Polyquaternium-10 | 0,20% | --- | 0,20% | --- | --- |
| Konservierungsmittel | 0,65% | 0,65% | 0,65% | 0,65% | 0,65% |
| Citronensäure | 0,50% | 0,50% | 0,50% | 1,00% | 0,75% |
| Parfum | q.s. | q.s. | q.s. | q.s. | q.s. |
| Wasser | ad 100 | ad 100 | ad 100 | ad 100 | ad 100 |
| Der pH-Wert wird auf 4,8-5,4 eingestellt. | | | | | |

### Kammer 1 (Duschformulierung) Beispiele 6-10:

| | **6** | **7** | **8** | **9** | **10** |
|---|---|---|---|---|---|
| Natrium Myrethsulfat | 5% | 4% | 6% | 8% | 7% |
| Laurylglucosid | 2,5% | --- | --- | --- | --- |
| Decylglucosid | --- | 3% | --- | 1,0% | 1,5% |
| Natrium Cocoamphoacetat | 6,5% | 7% | 8% | 4% | 3,5% |
| PEG-200 hydriertes Glycerylpalmitat | 0,40% | 0,40% | 0,40% | 0,30% | 0,35% |
| PEG-40 hydriertes Rizinusöl | 1 % | 1 % | 1 % | 1 % | 1 % |
| Diammonium Citrat | 0,12% | 0,12% | 0,12% | 0,12% | 0,12% |
| Polyquaternium-10 | 0,20% | --- | --- | 0,20% | --- |
| Konservierungsmittel | 0,5% | 0,5% | 0,5% | 0,5% | 0,5% |
| Citronensäure | 1,2% | 1,2% | 1,2% | 1,2% | 1,2% |
| Parfum | q.s. | q.s. | q.s. | q.s. | q.s. |
| Wasser | ad 100 | ad 100 | ad 100 | ad 100 | ad 100 |

### Kammer 2 (Shampoo) Beispiele 1-5:

| | **1** | **2** | **3** | **4** | **5** |
|---|---|---|---|---|---|
| Natrium Laurylethersulfat | 9 | 9 | 9 | 9 | 9 |
| Cocamidopropyl Betain | 4 | 4 | 4 | 4 | 4 |
| Dinatrium PEG-5 Laurylcitrat Sulfosuccinat | 3 | 3 | 3 | 3 | 3 |
| PEG-200 hydriertes Glycerylpalmitat | 0.1 | 0.1 | 0.1 | 0.2 | 0,3 |
| Polyquaternium-10 | 0.3 | 0.1 | 0.1 | 0.3 | - |
| Guar Hydroxypropyl-Trimonium Chlorid | - | - | 0.1 | 0.2 | 0.2 |
| Perlglanz | 1.5 | 3 | 4 | 2 | 2,5 |
| Trübungsmittel | - | - | - | - | 0.5 |
| Iminodibersteinsäure | 0.1 | 0.2 | 0.1 | 0.5 | 0.5 |
| PEG-40 hydriertes Rizinusöl | 0.2 | 0.2 | 0.2 | 0.2 | 0.2 |
| Konservierungsmittel | 0.8 | 0.8 | 0.8 | 0.8 | 0.8 |
| Natriumchlorid | 0,9 | 1,0 | 1,2 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

### Beispiel 3. Heating Effect

### Kammer 1: Tensidformulierung; Kammer 2: Tensid-freie Phase

### Kammer 1 (Tensidformulierung)

| | **1** | **2** | **3** | **4** | **5** |
|---|---|---|---|---|---|
| Natrium Laurethsulfat | 13,2% | 11% | 10% | 11% | --- |
| Natrium Myrethsulfat | --- | --- | --- | --- | 7,0% |
| Cocoamidopropylbetain | 1,65% | 3,3% | 1,5% | 3,3% | --- |
| Natriumcocoylglutamat | 1,25% | 0,75% | --- | 0,75% | --- |
| Natrium Cocoamphoacetat | --- | --- | --- | --- | 4,5% |
| Decylglucosid | --- | --- | --- | --- | 2,5% |
| Acrylat Copolymer | 0,5% | --- | --- | 0,7% | --- |
| Polyquaternium-22 | --- | --- | 0,2%% | --- | --- |
| Polyquaternium-10 | --- | --- | --- | --- | 0,3% |
| PEG-40 hydriertes Rizinusöl | 0,50% | 0,50% | 0,50% | 0,50% | 1,0% |
| PEG-100 hydriertes Glycerylpalmitat | 0,50% | 0,50% | 0,50% | 0,50% | --- |
| PEG-200 hydriertes Glycerylpalmitat | --- | --- | --- | --- | 0,4% |
| Diammonium Citrat | --- | --- | --- | --- | 0,12% |
| Natriumbenzoat | 0,45% | 0,45% | 0,45% | 0,45% | 0,3% |
| Natriumsalicylat | 0,20% | 0,20% | 0,20% | 0,20% | 0,2% |
| Citronensäure | 0,50% | 0,50% | 0,50% | 0,50% | 1,2% |
| Parfum | q.s. | q.s. | q.s. | q.s. | q.s. |
| Wasser | ad 100 | ad 100 | ad 100 | ad 100 | ad 100 |

### Kammer 2 (Tensid freie Phase)

| | **1** | **2** | **3** | **4** | **5** |
|---|---|---|---|---|---|
| Polyethylenglykol (Mw: 200-600) | 90% | 95% | 98% | 85% | 75% |
| Glycerin | 4% | 5% | --- | 5% | 7% |
| Diglycerin | 2% | --- | 2% | --- | --- |
| Polypropylenglycol | 4% | --- | --- | 10% | 7% |

### Beispiel 4. Gelförmige Duschzubereitungen mit Schwebepartikeln

### Kammer 1: Tensidphase; Kammer 2: Tensid freie Gelphase mit Schwebepartikeln

### Kammer 1 (Tensidformulierung (Beispiele 1-5)

| | **1** | **2** | **3** | **4** | **5** |
|---|---|---|---|---|---|
| Natrium Laurethsulfat | 13,2% | 11 % | 10% | 11 % | --- |
| Natrium Myrethsulfat | --- | --- | --- | --- | 7,0% |
| Cocoamidopropylbetain | 1,65% | 3,3% | 1,5% | 3,3% | --- |
| Natriumcocoylglutamat | 1,25% | 0,75% | --- | 0,75% | --- |
| Natrium Cocoamphoacetat | --- | --- | --- | --- | 4,5% |
| Decylglucosid | --- | --- | --- | --- | 2,5% |
| Acrylat Copolymer | 0,5% | --- | --- | 0,7% | --- |
| Polyquaternium-22 | --- | --- | 0,2% | --- | --- |
| Polyquaternium-10 | --- | --- | --- | --- | 0,3% |
| PEG-40 hydriertes Rizinusöl | 0,50% | 0,50% | 0,50% | 0,50% | 1,0% |
| PEG-100 hydriertes Glycerylpalmitat | 0,50% | 0,50% | 0,50% | 0,50% | --- |
| PEG-200 hydriertes Glycerylpalmitat | --- | --- | --- | --- | 0,4% |
| Diammonium Citrat | --- | --- | --- | --- | 0,12% |
| Natriumbenzoat | 0,45% | 0,45% | 0,45% | 0,45% | 0,3% |
| Natriumsalicylat | 0,20% | 0,20% | 0,20% | 0,20% | 0,2% |
| Citronensäure | 0,50% | 0,50% | 0,50% | 0,50% | 1,2% |
| Parfüm | q.s. | q.s. | q.s. | q.s. | q.s. |
| Wasser | ad 100 | ad 100 | ad 100 | ad 100 | ad 100 |

### Kammer 2 (Tensid freie Gelphase) (Beispiele 1-5)

| | **1** | **2** | **3** | **4** | **5** |
|---|---|---|---|---|---|
| Xanthan Gummi | 0,3% | - | 0,1 % | - | - |
| Acrylat Copolymer | - | 0,2% | - | 0,25% | - |
| Gellan Gummi | - | - | 0,2% | - | - |
| Acrylat/C10-C30 Alkylacrylat Copolymer | - | - | - | - | 0,25% |
| Natriumbenzoat | 0,3% | 0,3% | 0,3% | 0,3% | 0,3% |
| Natriumsalicylat | 0,2% | 0,2% | 0,2% | 0,2% | 0,2% |
| Natronlauge (15%ig) | - | 0,1% | - | 0,12% | 0,13% |
| Polyethylen Kugeln | 2% | - | - | - | 1 % |
| Walnußschalen Pulver | - | 3% | - | - | - |
| Cosmospheres (Fa. Pelletech) | - | - | 2% | - | - |
| Primaspheres (Fa. Cognis) | - | - | - | 4% | 1,5% |
| Parfum | q.s. | q.s. | q.s. | q.s. | q.s. |
| Wasser | ad 100 | ad 100 | ad 100 | ad 100 | ad 100 |

### Beispiel 5. Styling Gele mit Schwebepartikeln

### Kammer 1: Styling Gel; Kammer 2: Schwebepartikel

### Kammer 1 (Styling Gel) (Beispiele 1-5)

| | **1** | **2** | **3** | **4** | **5** |
|---|---|---|---|---|---|
| PVP/VA Copolymer | 5,0 % | 5,0 % | 4,5 % | 5,5 % | 4,0 % |
| Carbomer | 0,7 % | 1,0 % | 1,25 % | 0,9 % | 1,5 % |
| Parfum, Lösungsvermittler, Pflegestoffe | q.s. | q.s. | q.s. | q.s. | q.s. |
| Neutralisation/pH-Einstellung | q.s. | q.s. | q.s. | q.s. | q.s. |
| Ethanol | 10,0 % | --- | 11,0 % | 9,5 % | --- |
| Wasser | ad 100 | ad 100 | ad 100 | ad 100 | ad 100 |

### Kammer 2 (Gel-Phase mit Schwebepartikeln) (Beispiele 1-5)

| | **1** | **2** | **3** | **4** | **5** |
|---|---|---|---|---|---|
| Xanthan Gummi | 0,3% | - | 0,1% | - | - |
| Acrylat Copolymer | - | 0,2% | - | 0,25% | - |
| Gellan Gummi | - | - | 0,2% | - | - |
| Acrylat/C10-C30 Alkylacrylat Copolymer | - | - | - | - | 0,25% |
| Natriumbenzoat | 0,3% | 0,3% | 0,3% | 0,3% | 0,3% |
| Natriumsalicylat | 0,2% | 0,2% | 0,2% | 0,2% | 0,2% |
| Natronlauge (15%ig) | - | 0,1% | - | 0,12% | 0,13% |
| Polyethylen Kugeln | 2% | - | - | - | 1 % |
| Walnußschalen Pulver | - | 3% | - | - | - |
| Cosmospheres (Fa. Pelletech) | - | - | 2% | - | - |
| Primaspheres (Fa. Cognis) | - | - | - | 4% | 1,5% |
| Parfum | q.s. | q.s. | q.s. | q.s. | q.s. |
| Wasser | ad 100 | ad 100 | ad 100 | ad 100 | ad 100 |

### Beispiel 6.

### Kammer 1: Duschemulsion; Kammer 2: Duschformulierung

### Kammer 1 (Duschemulsion):

| | **1** | **2** | **3** | **4** | **5** |
|---|---|---|---|---|---|
| Paraffinöl | 46% | 14% | 20% | 20% | 25% |
| Sojaöl | 24,3% | 36% | 20% | 20% | 25% |
| Natriumlaurylethersulfat | 7,35% | 12,3% | 11% | 11% | 11% |
| Konservierungsmittel | 0,5% | 0,5% | 0,5% | 0,5% | 0,5% |
| Acrylates/C10-C30 Alkyl Acrylate Crosspolymer | - | - | 1% | 1% | 0,8% |
| Natriumhydroxid | - | - | 0,2% | 0,2% | 0,2% |
| Parfum | q.s. | q.s. | q.s. | q.s. | q.s. |
| Wasser | ad 100 | ad 100 | ad 100 | ad 100 | ad 100 |

### Kammer 2: Duschformulierung

| | **1** | **2** | **3** | **4** | **5** |
|---|---|---|---|---|---|
| Natrium Laurethsulfat | 13,2% | 11 % | 9,5% | 12,0% | 8,7% |
| Cocoamidopropylbetain | 1,65% | 3,3% | 3,8% | 4,5% | 2,5% |
| Natriumcocoylglutamat | 1,25% | 0,75% | 2,5% | 1,5% | 1,75% |
| PEG-40 hydriertes Rizinusöl | 0,50% | 0,50% | 0,50% | 0,50% | 0,50% |
| PEG-100 hydriertes Glycerylpalmitat | 0,50% | 0,50% | 0,50% | 0,65% | 0,50% |
| Polyquaternium-10 | 0,20% | --- | 0,20% | --- | --- |
| Konservierungsmittel | 0,65% | 0,65% | 0,65% | 0,65% | 0,65% |
| Citronensäure | 0,50% | 0,50% | 0,50% | 1,00% | 0,75% |
| Parfum | q.s. | q.s. | q.s. | q.s. | q.s. |
| Wasser | ad 100 | ad 100 | ad 100 | ad 100 | ad 100 |
| Der pH-Wert wird auf 4,8-5,4 eingestellt. | | | | | |

### Kammer 2 (Duschformulierung) Beispiele 6-10:

| | **6** | **7** | **8** | **9** | **10** |
|---|---|---|---|---|---|
| Natrium Myrethsulfat | 5% | 4% | 6% | 8% | 7% |
| Laurylglucosid | 2,5% | --- | --- | --- | --- |
| Decylglucosid | --- | 3% | --- | 51,0% | 1,5% |
| Natrium Cocoamphoacetat | 6,5% | 7% | 8% | 4% | 3,5% |
| PEG-200 hydriertes Glycerylpalmitat | 0,40% | 0,40% | 0,40% | 0,30% | 0,35% |
| PEG-40 hydriertes Rizinusöl | 1 % | 1 % | 1 % | 1 % | 1 % |
| Diammonium Citrat | 0,12% | 0,12% | 0,12% | 0,12% | 0,12% |
| Polyquaternium-10 | 0,20% | --- | --- | 0,20% | --- |
| Konservierungsmittel | 0,5% | 0,5% | 0,5% | 0,5% | 0,5% |
| Citronensäure | 1,2% | 1,2% | 1,2% | 1,2% | 1,2% |
| Parfum | q.s. | q.s. | q.s. | q.s. | q.s. |
| Wasser | ad 100 | ad 100 | ad 100 | ad 100 | ad 100 |

### Beispiel 7. Post foam Gel auf Basis von Carbonaten

### Kammer 1: Tensid Phase; Kammer 2: Carbonat haltige Phase

### Kammer 1 (Tensidformulierung) (Beispiele 1-5)

| | **1** | **2** | **3** | **4** | **5** |
|---|---|---|---|---|---|
| Natrium Laurethsulfat | 13,2% | 11 % | 10% | 11 % | --- |
| Natrium Myrethsulfat | --- | --- | --- | --- | 7,0% |
| Cocoamidopropylbetain | 1,65% | 3,3% | 1,5% | 3,3% | --- |
| Natriumcocoylglutamat | 1,25% | 0,75% | --- | 0,75% | --- |
| Natrium Cocoamphoacetat | --- | --- | --- | --- | 4,5% |
| Decylglucosid _ | --- | --- | --- | --- | 2,5% |
| Acrylat Copolymer | 0,5% | --- | --- | 0,7% | --- |
| Polyquaternium-22 | --- | --- | 0,2%% | --- | --- |
| Polyquaternium-10 | --- | --- | --- | --- | 0,3% |
| PEG-40 hydriertes Rizinusöl | 0,50% | 0,50% | 0,50% | 0,50% | 1,0% |
| PEG-100 hydriertes Glycerylpalmitat | 0,50% | 0,50% | 0,50% | 0,50% | --- |
| PEG-200 hydriertes Glycerylpalmitat | --- | --- | --- | --- | 0,4% |
| Diammonium Citrat | --- | --- | --- | --- | 0,12% |
| Natriumbenzoat | 0,45% | 0,45% | 0,45% | 0,45% | 0,3% |
| Natriumsalicylat | 0,20% | 0,20% | 0,20% | 0,20% | 0,2% |
| Citronensäure | 0,50% | 0,50% | 0,50% | 0,50% | 1,2% |
| Parfum | q.s. | q.s. | q.s. | q.s. | q.s. |
| Wasser | ad 100 | ad 100 | ad 100 | ad 100 | ad 100 |
| Der pH Wert wird in einem Bereich von 2,5-4 eingestellt | | | | | |

### Kammer 2 (Tensid freie Gelphase) (Beispiele 1-5)

| | **1** | **2** | **3** | **4** | **5** |
|---|---|---|---|---|---|
| Xanthan Gummi | 0,3% | - | 0,1% | - | - - |
| Acrylat Copolymer | - | 0,2% | - | 0,25% | - |
| Gellan Gummi | - | - | 0,2% | - | - |
| Acrylat/C10-C30 Alkylacrylat Copolymer | - | - | - | - | 0,25% |
| Natriumbenzoat | 0,3% | 0,3% | 0,3% | 0,3% | 0,3% |
| Natriumsalicylat | 0,2% | 0,2% | 0,2% | 0,2% | 0,2% |
| Natronlauge (15%ig) | - | 0,1% | - | 0,12% | 0,13% |
| Carbonate | 7,0 % | 15 % | 1,5 % | 2,0 % | 10%- |
| Parfum | q.s. | q.s. | q.s. | q.s. | q.s. |
| Wasser | ad 100 | ad 100 | ad 100 | ad 100 | ad 100 |
| Der pH Wert wird auf einen Bereich von 7,5-9 eingestellt. | | | | | |

### Beispiel 8. Kosmetisches Reinigungsmittel für unterschiedliche Körperzonen

### Kammer 1: stark schäumendes Duschgel; Kammer 2: milder Gesichtsreiniger

### Kammer 1: Stark schäumendes Duschgel

| | **1** | **2** | **3** | **4** | **5** |
|---|---|---|---|---|---|
| Natrium Laurethsulfat | 13,2% | 11% | 9,5% | 12,0% | 8,7% |
| Cocoamidopropylbetain | 1,65% | 3,3% | 3,8% | 4,5% | 2,5% |
| Natriumcocoylglutamat | 1,25% | 0,75% | 2,5% | 1,5% | 1,75% |
| PEG-40 hydriertes Rizinusöl | 0,50% | 0,50% | 0,50% | 0,50% | 0,50% |
| PEG-100 hydriertes Glycerylpalmitat | 0,50% | 0,50% | 0,50% | 0,65% | 0,50% |
| Polyquaternium-10 | 0,20% | --- | 0,20% | --- | --- |
| Konservierungsmittel | 0,65% | 0,65% | 0,65% | 0,65% | 0,65% |
| Citronensäure | 0,50% | 0,50% | 0,50% | 1,00% | 0,75% |
| Parfum | q.s. | q.s. | q.s. | q.s. | q.s. |
| Wasser | ad 100 | ad 100 | ad 100 | ad 100 | ad 100 |

### Kammer 2: milder Gesichtsreiniger

| | **1** | **2** | **3** | **4** | **5** |
|---|---|---|---|---|---|
| Natrium Laurethsulfat | 1,5 % | 2,0 % | 1,0 % | 3,0 % | 0,5 % |
| Decylglucosid | 0,5 % | 0,75 % | 1,0 % | 1,5 % | 2,0 % |
| Glycerin | 2,8 % | 1,5 % | 1,0 % | 3,0 %. | 1,75 % |
| Stabilisator | 0,09 % | 0,04 % | 0,1 % | 0,02 % | 0,05 % |
| Carbomer | 2,0 % | 1,50 % | 1,0 % | 0,50 % | 0,75 % |
| Xanthan Gum | 0,35 % | 0,20 % | 0,15 % | 0,35 % | 0,45 % |
| PEG-7-Glyceryl Cocoate | 0,50 % | 0,25 % | 0,30 % | 0,15 % | 0,20 % |
| Sodium Methyl Cocoyl Taurat | 0,75 % | 0,25 % | 0,50 % | 0,30 % | 1,00 % |
| Moisturizer | 0,50 % | 0,25 % | 0,40 % | 1,0% | 0,75 % |
| Farbstoff | q.s. | q.s. | q.s. | q.s. | q.s. |
| Konservierungsmittel | 0,65% | 0,55% | 0,15% | 0,25% | 0,35% |
| Natronlauge | 0,45 % | 0,50 % | 0,65 % | 0,35 % | 0,45 % |
| Parfum | q.s. | q.s. | q.s. | q.s. | q.s. |
| Wasser | ad 100 | ad 100 | ad 100 | ad 100 | ad 100 |

## Patentansprüche

1. Kosmetikum, welches gebildet wird aus
a) einem Packmittel mit zwei von einander getrennten Vorratskammern, deren Inhalt mit Hilfe zweier gekoppelter Förderpumpen gleichzeitig über eine gemeinsame Austrittsöffnung entnommen werden kann und **dadurch gekennzeichnet ist, dass** sich das Mengenverhältnis der durch die beiden Pumpen geförderten kosmetischen Zubereitungen zueinander mit Hilfe eines Regelmechanismus frei einstellen lässt, sowie
b) zwei kosmetischen Zubereitungen 1 und 2, die sich jeweils in einer der beiden Vorratskammern des Packmittels befinden und **dadurch gekennzeichnet sind, dass** sie sich in ihrer Viskosität und/oder Konzentration an kosmetischen und/oder dermatologischen Wirkstoffen von einander unterscheiden.

2. Verwendung eines Packmittels mit zwei von einander getrennten Vorratskammern, deren Inhalt mit Hilfe zweier gekoppelter Förderpumpen gleichzeitig über eine gemeinsame Austrittsöffnung entnommen werden kann und **dadurch gekennzeichnet ist, dass** sich das Mengenverhältnis der durch die beiden Pumpen geförderten kosmetischen Zubereitungen zueinander mit Hilfe eines Regelmechanismus frei einstellen lässt, zur variablen Einstellung des Mischungsverhältnisses zweier kosmetischer Zubereitungen unterschiedlicher Viskosität.

3. Verwendung eines Packmittels mit zwei von einander getrennten Vorratskammern, deren Inhalt mit Hilfe zweier gekoppelter Förderpumpen gleichzeitig über eine gemeinsame Austrittsöffnung entnommen werden kann und **dadurch gekennzeichnet ist, dass** sich das Mengenverhältnis der durch die beiden Pumpen geförderten kosmetischen Zubereitungen zueinander mit Hilfe eines Regelmechanismus frei einstellen lässt, zur Steigerung der Wirksamkeit kosmetischer Zubereitungen und der in ihnen enthaltenen, Wirkstoffe, welche durch die getrennte Aufbewahrung einzelner Teilzubereitungen und deren gleichzeitiger Applizierung in variabler Dosierung bewirkt wird.

4. Verwendung eines Packmittels mit zwei von einander getrennten Vorratskammern, deren Inhalt mit Hilfe zweier gekoppelter Förderpumpen gleichzeitig über eine gemeinsame Austrittsöffnung entnommen werden kann und **dadurch gekennzeichnet ist, dass** sich das Mengenverhältnis der durch die beiden Pumpen geförderten kosmetischen Zubereitungen zueinander mit Hilfe eines Regelmechanismus frei einstellen lässt, zur individuellen Wirkstoffdosierung wirkstoffhaltiger kosmetischer oder dermatologischer Zubereitungen.

5. Kosmetikum nach Anspruch 1 oder Verwendung nach einem der Ansprüche.2 bis 4, **dadurch gekennzeichnet, dass** die variable Einstellung des Mengenverhältnisses dadurch erfolgt, dass der mit Hilfe eines gemeinsamen Pumpauslösers erzeugte Druck auf die beiden Förderpumpen mit Hilfe einer drehbaren Scheibe unterschiedlich stark auf die beiden Pumpenköpfe verteilt wird.

6. Kosmetikum oder Verwendung nach einem der vorhergehenden Ansprüche, **dadurch gekennzeichnet, dass** der Viskositäts-Unterschied zwischen den beiden kosmetischen Zubereitungen 1 und 2 größer als 50 mPas und kleiner als 3000 mPas ist.

7. Kosmetikum oder Verwendung nach einem der vorhergehenden Ansprüche, **dadurch gekennzeichnet, dass** der Konzentrationsunterschied an einem oder mehreren bestimmten kosmetischen und/oder dermatologischen Wirkstoffen in den kosmetischen Zubereitungen 1 und 2 mindestens 0,01 % beträgt.

8. Kosmetikum oder Verwendung nach einem der vorhergehenden Ansprüche, **dadurch gekennzeichnet, dass** die kosmetische Zubereitung 1 einen oder mehrere Inhaltsstoffe enthält, die aus der Gruppe der anionischen Tenside, nichtionischen Tenside, amphoteren Tenside, Betaine, ethoxylierten Mono-, di- und Triglyceriden, anionischen, nichtionischen und amphoteren Polymeren, Parfümölen, Enzymsubstraten, Konditionierer, Emulgatoren Lipiden und Silikonölen gewählt werden können.

9. Kosmetikum oder Verwendung nach einem der vorhergehenden Ansprüche, **dadurch gekennzeichnet, dass** die kosmetische Zubereitung 2 einen oder mehrere Inhaltsstoffe enthält, die aus der Gruppe der wasserlöslichen Wirkstoffe, kationischen Tenside, kationischen Poymeren, Emulgatoren und Farbstoffen gewählt werden können.
